# EUROPEAN PATENT SPECIFICATION

(11) **EP 2 601 982 B1**
(45) Date of publication and mention of the grant of the patent: **08.04.2015**
(21) Application number: 11814841.0
(22) Date of filing: 05.08.2011
(51) Int. Cl.: A61L 27/36, A61F 2/28, C12N 5/071, A61L 2/025, A61L 27/38, A61L 27/58

(54) **METHOD FOR PROCESSING BONE GRAFT MATERIAL USING TEETH, AND BONE GRAFT MATERIAL PROCESSED THEREBY**
VERFAHREN ZUR BEARBEITUNG EINES KNOCHENERSATZMATERIALS UNTER VERWENDUNG VON ZÄHNEN UND AUF DIESE WEISE BEARBEITETES KNOCHENERSATZMATERIAL
PROCÉDÉ DE TRAITEMENT D'UN MATÉRIAU POUR GREFFE OSSEUSE UTILISANT DES DENTS, ET MATÉRIAU POUR GREFFE OSSEUSE AINSI TRAITÉ

(30) Priority: 05.08.2010 KR 20100075400
(43) Date of publication of application: 12.06.2013
(73) Proprietor: Um, In Woong, Seoul 136-044 (KR)
(72) Inventor: Um, In Woong, Seoul 136-044 (KR)
(74) Representative: ABG Patentes, S.L.
(86) International application number: PCT/KR2011/005748
(87) International publication number: WO 2012/018241

(56) References cited:
- WO-A1-2004/089432
- JP-A- H04 114 656
- KR-A- 20020 011 164
- KR-A- 20040 065 203
- KR-A- 20040 087 438
- KR-A- 20100 040 427
- KIM Y K ET AL: "Development of a novel bone grafting material using autogenous teeth", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 109, no. 4, 1 April 2010 (2010-04-01) , pages 496-503, XP026968408, ISSN: 1079-2104 [retrieved on 2010-01-08]
- DATABASE WPI Week 201028 Thomson Scientific, London, GB; AN 2010-E35484 XP002718614, & WO 2010/041814 A2 (KOREA TISSUE BANK CO LTD) 15 April 2010 (2010-04-15)

## Description

### TECHNICAL FIELD

The present invention relates to a bone graft material, and more particularly, a method for processing a bone graft material using teeth by dividing an extracted tooth by each region and processing the extracted teeth to allow regional powder to be independently used or mixed depending on the use for alveolar bone graft or alveolar reinforcement, and a graft material processed thereby.

### BACKGROUND ART

Recent trend of tooth implantation is immediate placement after extraction, and the preservation and ridge augmentation of alveolar are indispensable.

The bone graft materials used in the preservation and augmentation of alveolar ridge for implantation include synthetic bones, xenografts and allografts. The synthetic bones, xenografts and allografts do not surpass autogenous bone grafts. Most of the currently used graft materials are inorganic matter, lipoid or partially synthesized materials of autogenous bones for the purpose of performing a part of the functions of the autogenous bones.

In addition, the fixture for an implant must be fixed rigidly, however, it is not easy for the patients having native or acquired defect in the alveolar to fix the fixture rigidly. In such cases, conventional implantation methods require transplantation of other person's bone or a bone graft material made of artificial structure to the alveolar to treat the native or acquired defect before implantation.

However, the bone graft materials from another person have the difficulty of immunity rejection response and complicated treatment process to solve the immunity issue.

In addition, the bone graft materials made of artificial structure have low generation rates of alveolar since the materials do not contain bone morphogenetic proteins.

Using autogenous bone graft materials to solve the said problems also requires additional operation and the supply is limited.

As such, it is necessary to develop bone graft materials which may solve above mentioned problems.

Bone grafts based on dental materials are already known in the art such as in WO 2004/089432 or in the article from Young-Kuyn Kim et al. (Oral surg. oral med. oral pathol. oral radiol endod, 2010, 109,496-503) but no specific selection of part of the starting material has been investigated in those documents.

### DISCLOSURE OF THE INVENTION

### TECHNICAL PROBLEM

To solve above-described problems, the present invention provides a method for producing a bone graft material by processing extracted tooth into a bone graft material having the same osteoinduction and osteoconduction as autogenous bones.

The present invention also provides a bone graft material having at least one of osteoinduction and osteoconduction processed by the bone graft material processing method describe above.

The present invention also provides a method for processing a bone graft material using a tooth having the same functionalities and effectiveness of autogenous bone graft material, solving the immunity issue and providing good alveolar generation rate, and the bone graft material processed thereby.

### TECHNICAL SOLUTION

The bone graft material processing method using a tooth in accordance with the present invention includes the steps of: (a) cutting a tooth extracted from a patient at the boundary between the dental crown and the dental root of the tooth; (b) removing the missing region of the dental crown cut, soft tissue and pulp tissue; (c) quick-freezing the dental crown and pulverizing the same to prepare a powder having an average diameter of about 200 µm to about 1,500 µm; (d) degreasing the powder; (e) deliming the powder, (f) dehydrating the powder; (g) degreasing the powder once again; and (h) sterilizing the degreased and dehydrated powder to prepare bone graft materials.

In addition, the bone graft material processing method using a tooth in accordance with the present invention may further include the step of washing the dental crown of the tooth with distilled water for about 30 minutes to 2 hours after the step (b).

In addition, the quick-freezing process of the step (c) may be performed to freeze the dental crown in liquid nitrogen at a temperature between about -160°C to - 210°C for about 30 minutes to about 2 hours.

In addition, the bone graft material processing method using a tooth in accordance with the present invention may further include the step of removing contaminants and residual soft tissues from the powder prepared in the step (c) by washing the powder with distilled water for about 30 minutes to about 2 hours.

In addition, the powder of the step (c) may be processed by three cycles of ultrasonic cleaning of which each cycle includes: a first washing step where the powder is processed with ultrasonic cleaning for about 5 minutes to about 10 minutes in pure water or sterilized distilled water; a second washing step where the powder is processed with ultrasonic cleaning for about 10 minutes to about 30 minutes in hydrogen peroxide solution; and a third washing step where the powder is processed with ultrasonic cleaning for about 5 minutes to about 10 minutes in pure water or sterilized distilled water, wherein the concentration of the hydrogen peroxide solution may be about 5% to about 7 %, the volume of the washing fluid of each washing step may be between about 5 times to about 10 times that of the powder, and the temperature of the washing fluid may range between about 60°C to about 80°C.

In addition, the degreasing step (d) may be performed using chloroform methanol solution where the ratio between the chloroform and methanol is about 1:0.5 to about 1:2 by weight, for 3 hours to 12 hours.

In addition, the bone graft material processing method using a tooth in accordance with the present invention may further include the steps of removing the floating fat from the powder by centrifugal separation after degreasing of the step (d); and washing the powder with distilled water for about 1 hour to about 2 hours after the centrifugal separation.

In addition, the dehydration of the step (f) may be performed for about 30 minutes to about 2 hours using neutral ethyl alcohol.

In addition, the bone graft material processing method using a tooth in accordance with the present invention may further include the step of mixing an additive generated in the dental root and having higher osteoinduction with the powder, before the step (h).

In addition, the bone graft material processing method using a tooth in accordance with the present invention may further include the powder processed from the dental root by removing soft tissue, dehydrating, degreasing and deliming.

The bone graft material processing method using a tooth in accordance with the present invention includes the steps of: (a) cutting a tooth extracted from a patient at the boundary between the dental crown and the dental root of the tooth; (b) removing the soft tissue from the dental root cut; (c) quick-freezing the dental root and pulverizing the same to prepare powder having an average diameter of about 200 µm to about 1,500 µm; (d) degreasing the powder; (e) deliming the powder; (f) dehydrating the powder; (g) degreasing the powder once again; and (h) sterilizing the degreased and dehydrated powder to prepare a bone graft material.

In addition, the bone graft material processing method using a tooth in accordance with the present invention may further include the step of washing the dental root with distilled water for about 30 minutes to about 2 hours after the step (b).

In addition, the quick-freezing process of the step (c) may be performed to freeze the dental root in liquid nitrogen at a temperature between about -160°C to about -210°C for about 30 minutes to about 2 hours.

In addition, the bone graft material processing method using a tooth in accordance with the present invention may further include the step of removing contaminants and residual soft tissues from the powder prepared in the step (c) by washing the powder with distilled water for about 30 minutes to about 2 hours.

In addition, the powder of the step (c) may be processed by three cycles of ultrasonic cleaning of which each cycle includes: a first washing step where the powder is processed with ultrasonic cleaning for about 5 minutes to about 10 minutes in pure water or sterilized distilled water; a second washing step where the powder is processed with ultrasonic cleaning for about 10 minutes to about 30 minutes in hydrogen peroxide solution; and a third washing step where the powder is processed with ultrasonic cleaning for about 5 minutes to about 10 minutes in pure water or sterilized distilled water, wherein the concentration of the hydrogen peroxide solution may be about % 5 to about 7 %, the volume of the washing fluid of each washing step may be between about 5 times to 10 times that of the powder, and the temperature of the washing fluid may be between about 60°C to about 80°C.

In addition, the degreasing steps (d) and (g) may be performed using chloroform methanol solution where the ratio between the chloroform and methanol is about 1:0.5 to about 1:2 by weight percent, for about 3 hours to about 12 hours.

In addition, the bone graft material processing method using a tooth in accordance with the present invention may further include the steps of removing the floating fat from the powder by centrifugal separation after degreasing of the step (d); and washing the powder with distilled water for one to two hours after the centrifugal separation.

In addition, the deliming of the step (e) may be performed using about 0.1 N to about 1.0 N hydrochloric acid aqueous solution having a volume of about 5 times to about 10 times that of the powder for about 10 minutes to about 1 hour.

In addition, the dehydration of the step (f) may be performed for about 30 minutes to about 2 hours using neutral ethyl alcohol.

In addition, the bone graft material processing method using a tooth in accordance with the present invention may further include the step of mixing an additive generated in the dental crown and having higher osteoconduction with the powder, before the step (h).

In addition, the additive may include the powder produced from the dental crown by removing soft tissue, dehydrating and degreasing.

In addition, the bone graft material using a tooth in accordance with the present invention may be processed by any one of the methods described above.

### ADVANTAGEOUS EFFECTS

The present invention may process extracted teeth into bone graft materials having the same osteoinduction and osteoconduction as those of autogenous bones, and the graft materials having the same functions and effectiveness as those of autogenous bones enable alveolar implantation with high alveolar regeneration rate solving the immunity issue.

### BRIEF DESCRIPTION OF THE DRAWINGS

FIG. 1 is a sectional view illustrating the structure of tooth to be processed in accordance with the present invention.
FIG. 2 is a picture of a tooth extracted for a bone graft material processing method using a tooth in accordance with the present invention.
FIG. 3 is a picture of the extracted tooth of FIG. 2 after removing the defective part such as dental caries and inflammation.
FIGS. 4 and 5 are pictures of the tooth cut at the boundary between the dental crown and dental root.
FIG. 6 is a picture of a tooth after removing soft tissue and pulp tissue.
FIG. 7 is a picture of a bone graft material prepared using a tooth in accordance with the method of the present invention, after quick-freezing and pulverizing.
FIG. 8 is a conceptual view of the dentin powder at the initial stage of implantation in accordance with the present invention.
FIGS. 9 and 10 are pictures of the implanted region of dentin powder in accordance with the present invention in the middle and latter periods.
FIG. 11 is a conceptual view of enamel powder at the initial stage of implantation in accordance with the present invention.

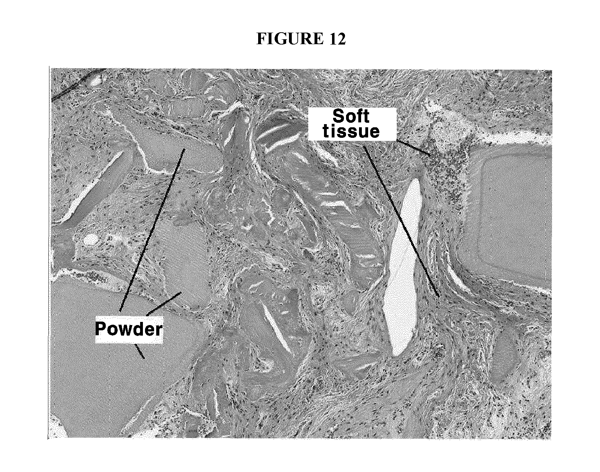

FIG. 12 is a picture of the implanted region of enamel powder in accordance with the present invention in the latter period.

### MODE FOR CARRYING OUT THE INVENTION

The present invention discloses a method for processing a tooth extracted from a patient into bone graft materials for alveolar, and the bone graft materials may be implanted into the patient or other patients.

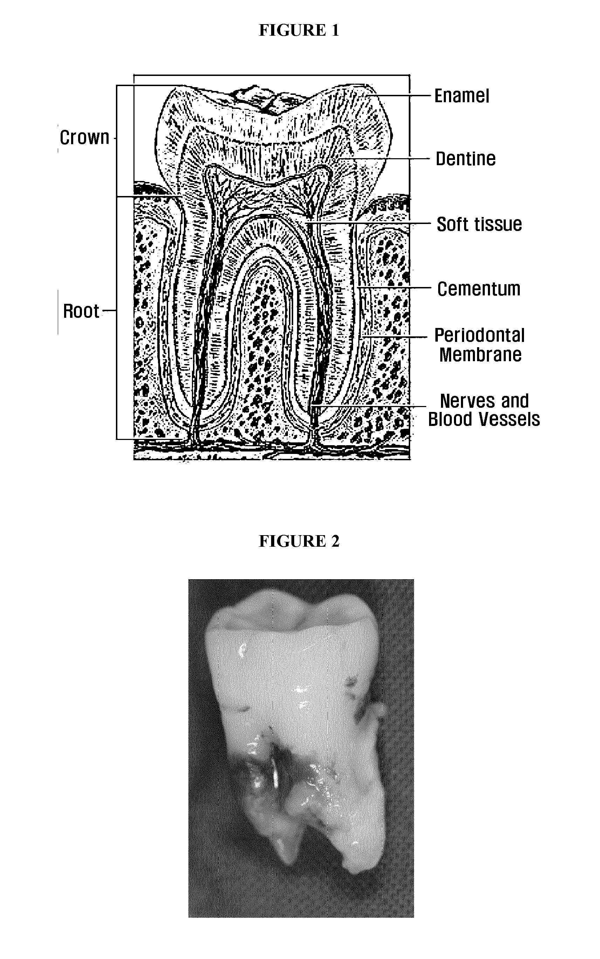

Referring to FIG. 1, the part of a tooth exposed out of the gingiva is called dental crown and the part inside the gingiva is called dental root. The components of a tooth may be classified into enamel, dentin, pulp, cementum, periodontal membrane and nerve and blood supply by the material.

The enamel which is a hard tissue covering and protecting a tooth on the surface consists of about 96% of inorganic materials. The dentin is the most similar tissue with the human bone tissue, a light-yellow or yellowish white, opaque tissue, softer than the enamel and harder than the cementum. The cementum which bonds the tooth to alveolar is a white, a little opaque tissue harder than bone tissue and softer than enamel. The pulp is a soft tissue at the center of a tooth, including nerve and blood suppy. The periodontal membrane is a tissue bonding the cementum of a tooth and alveolar.

Here, the enamel, dentin and cementum may be classified as hard tissues and the pulp, periodontal membrane, nerve and blood supply may be classified as soft tissues.

The tooth tissues described above are similar to those of autogenous bone but having different ingredients and physical properties according to the parts. The parts available for bone graft material include enamel, dentin and cementum. The enamel may be used as a void filler and graft expander having osteoconduction, and the dentin and cementum has osteoinduction and osteoconduction.

The tooth of FIG. 1 is cut horizontally at the boundary (Cementoenamel junction; CEJ) between the dental crown and dental root which are pulverized into powder. Since the powders from the dental crown and dental root differ in their compositions, they may be used as graft materials independently or as a mixture of the two. The Examples use the extracted tooth of FIG. 2, from which the defective part such as dental caries or inflammation preferably be removed before processing.

### EXAMPLE

### Example 1: Enamel Powder

An Example wherein the dental crown of an extracted tooth is processed into powder will be described below.

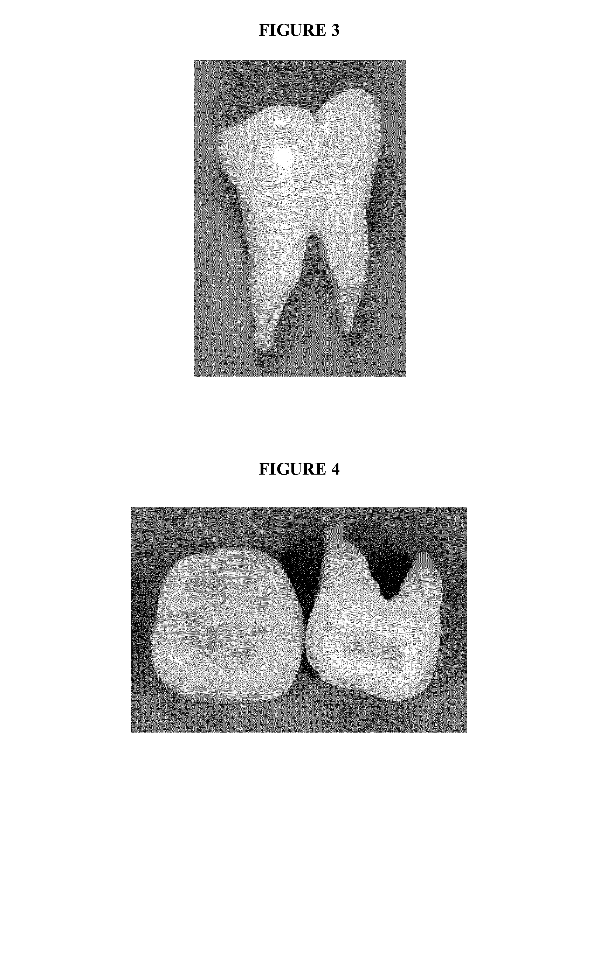

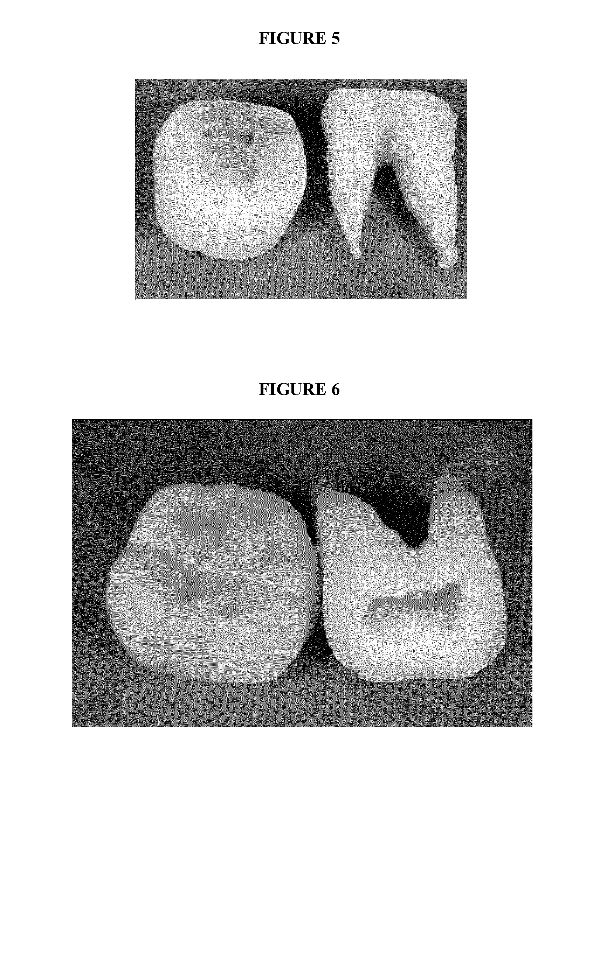

To begin with, the extracted tooth whose defective parts such as dental caries and inflammation are removed as shown on Fig. 3 is cut at the boundary between the dental crown and dental root, as shown on FIG. 4 and FIG. 5.

The dental crown whose soft and pulp tissues are removed, as shown in FIG. 6, is washed with distilled water for about 30 minutes to about two hours. The dental crown whose soft and pulp tissues are removed as shown in FIG. 6, may have both enamel and dentin.

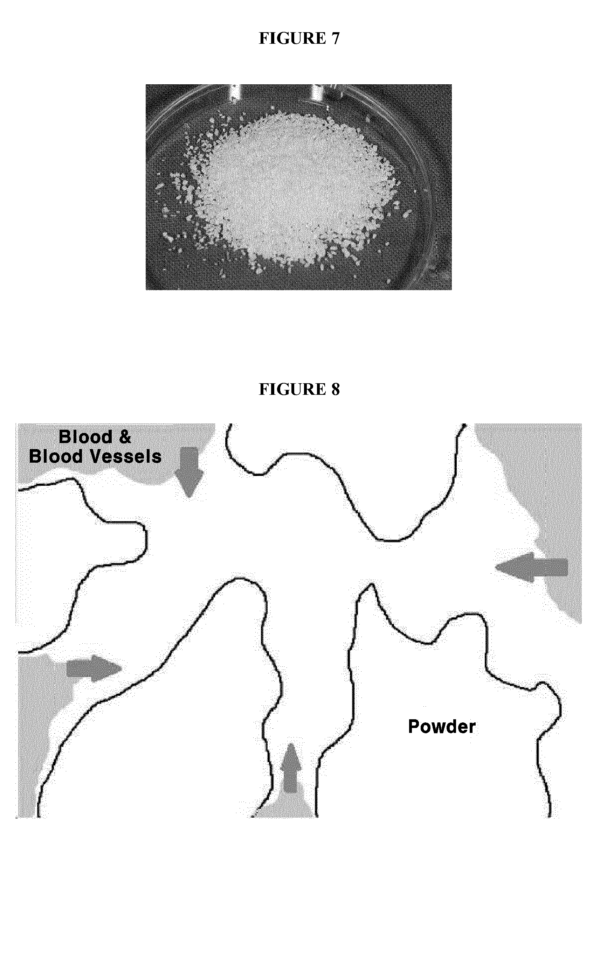

After the process, the treated dental crown as shown in FIG. 6 is quick-frozen in liquefied nitrogen at about -160°C to about -210°C for about 30 minutes to 2 hours, and crushed with a pulverizer into power whose average particle diameter ranges between from about 200 µm to about 1,500 µm, as shown in FIG. 7.

The powder may be washed with distilled water for about 30 minutes to about 2 hours to remove contaminants and residual soft tissue.

On the other hand, the powder may be processed by three cycles of ultrasonic cleaning of which each cycle includes: a first washing step where the powder is processed with ultrasonic cleaning for about 5 minutes to about 10 minutes in pure water or sterilized distilled water; a second washing step where the powder is processed with ultrasonic cleaning for about 10 minutes to about 30 minutes in hydrogen peroxide solution; and a third washing step where the powder is processed with ultrasonic cleaning for about 5 minutes to 10 minutes in pure water or sterilized distilled water, to remove contaminants and residual soft tissue. Here, the concentration of the hydrogen peroxide solution is about 5% to about 7 %, the volume of the washing fluid of each washing step is between about 5 times to 10 times that of the powder, and the temperature of the washing fluid may be between about 60°C to about 80°C

The powder washed in the above described the step may be degreased using chloroform methanol solution where the ratio between the chloroform and methanol is about 1:0.5 to about 1:2 by weight, for about 3 hours to about 12 hours.

The degreased powder may be get rid of floating fat by centrifugal separation and washed with distilled water for about 1 hour to about 2 hours.

The degreased powder described above is dehydrated with neutral ethyl alcohol for about 30 minutes to about 2 hours.

The powder degreased and dehydrated as described above is post-treated by the processes which comprise: the process of washing and freeze-drying the degreased and dehydrated powder; and the process of sterilizing the freeze-dried powder using radiation or ethylene oxide gas.

As described above, the powder containing enamel and dentin may have the osteoconduction of enamel and osteoinduction of dentin. However, since the content of the enamel is greater than that of the dentin and the deliming process for the dentin is not applied, the powder processed in accordance with the Example 1 has higher osteoconduction.

The powder produced from dental crown by degreasing and dehydrating processes as described above may be used as a bone graft material for alveolar implantation independently, or by being mixed with an additive, produced from the dental root, which may strengthen osteoinduction. Here, the additive may be the powder of Example 2 described below, produced from the dental root whose soft and pulp tissues are removed and dehydrated, degreased, and delimed.

### Example 2: Dentin Powder

An Example wherein the dental root of an extracted tooth is processed into powder will be described below.

To begin with, the extracted tooth whose defective parts such as dental caries and inflammation are removed as shown on Fig. 3 is cut at the boundary between the dental crown and dental root, as shown on FIG. 4 and FIG. 5.

The dental root whose soft and pulp tissues are removed, as shown in FIG. 6, is washed with distilled water for about 30 minutes to about 2 hours. As shown in FIG. 6, the dental root without soft and pulp tissues has residual dentin and cementum.

The dental root treated as shown in FIG. 6 is processed by quick-freezing and crushing, the same method applied to the dental crown as described in Example 1, into powder whose average particle diameter is about 200 µm to about 1,500 µm.

The powder may be washed with distilled water for about 30 minutes to about 2 hours to remove contaminants and residual soft tissue, same as Example 1.

Same as Example 1, the powder may be processed by three cycles of ultrasonic cleaning of which each cycle includes: a first washing step where the powder is processed with ultrasonic cleaning for about 5 minutes to about 10 minutes in pure water or sterilized distilled water; a second washing step where the powder is processed with ultrasonic cleaning for about 10 minutes to about 30 minutes in hydrogen peroxide solution; and a third washing step where the powder is processed with ultrasonic cleaning for about 5 minutes to about 10 minutes in pure water or sterilized distilled water, to remove contaminants and residual soft tissue. Here, the concentration of the hydrogen peroxide solution is about 5% to about 7 %, the volume of the washing fluid of each washing step is between about 5 times to about 10 times that of the powder, and the temperature of the washing fluid may be between about 60°C to 80°C.

The powder washed in the above described step may be primarily degreased using chloroform methanol solution where the ratio between the chloroform and methanol is about 1:0.5 to about 1:2 by weight, for about 3 hours to about 12 hours.

The primarily degreased powder may be get rid of floating fat by centrifugal separation and washed with distilled water for at least 1 hour.

The primarily degreased powder as described above is delimed with about 0.5N hydrochloric acid aqueous solution whose volume is about 5 times to about 10 times greater than that of the powder for about 10 minutes to about 1 hour.

Then, the delimed powder is dehydrated with neutral ethyl alcohol for about 30 minutes to about 2 hours.

The dehydrated powder may be secondarily degreased using chloroform methanol solution where the ratio between the chloroform and methanol is about 1:0.5 to about 1:2 by weight, for about 3 hours to about 12 hours.

The powder degreased, delimed and dehydrated as described above is post-treated by the processes which include the step of: washing with normal saline solution and freeze-drying the degreased, delimed and dehydrated powder; and the step of sterilizing the freeze-dried powder using radiation or ethylene oxide gas.

The delimed powder processed in the above described processes may contain residual calcium by about 8 % or less, and the usage of hydrochloric acid aqueous solution may be between about 2 ml to about 10 ml per gram of powder.

As described above, the powder produced from dentin and cementum in accordance with Example 2 of the present invention may have the osteoconduction and osteoinduction of dentin and cementum. However, the powder in accordance with Example 2 has higher osteoinduction than that of the powder in accordance with Example 1.

The powder produced from dental crown by degreasing, deliming and dehydrating processes as described above may be used as a bone graft material for alveolar implantation independently, or by being mixed with an additive, produced from the dental crown, which may strengthen osteoconduction. Here, the additive may be the powder of Example 1 produced from the dental crown whose soft and pulp tissues are removed and dehydrated, degreased, and delimed.

As described in Examples 1 and 2, bone graft materials may be obtained in powder. The bone graft materials processed in accordance with the present invention may have relatively higher osteoinduction or osteoconduction according to the raw material, and they may be used independently or in a mixture as the bone graft materials for alveolar.

More particularly, a molar tooth may produce from about 0.14 g to about 0.40 g of dentin (differs by prosthetic dentistry) and from about 0.10 g to about 0.78 g of enamel powder. The output may vary by prosthetic dentistry, and in average, a molar tooth may produce about 0.43 g of enamel and about 0.20 g of dentin.

The powder obtained in the Example 1 is called enamel powder and the powder obtained in Example 2 is called dentin powder.

The enamel powder has similar properties with the osteoconductive materials produced from the commercial synthetic bones and animal bones. However, the enamel of human tooth is superior in biocompatibility to the inorganic matter of conventional synthetic bones and features better absorbability and bone remodeling performance. As such, the product may maintain the volume and shape in the earlier stage and remodeled into bone similar with the alveolar bearing chewing force in the latter stage. Furthermore, since the powder contains dentin component which is difficult to be separated physically by about 1/3 to about 1/4, it also provides osteoinduction. Here, since the osteoinduction of the dentin which is not delimed is close to the osteoconduction without rapid decrease in volume or absorption.

Therefore, the enamel powder produced in accordance with Example 1 of the present invention is suitable for implantation out of alveolar, implantation at a position where blood supply is poor, or to obtain aesthetic volume over a long time.

The dentin power having relatively higher osteoinduction is advantageous for use in alveolar, where blood supply is well, or at the interface of implants to generate bones having higher activity. Since the dentin powder maynot maintain the initial volume due to fast osteoinduction and bone remodeling performance, it is suitable for implantation in alveolar.

For a newly-extracted tooth not requiring differentiation between enamel powder and dentin powder, the two kinds of powder may be used as a mixture. Or independently, dentine powder is suitable for the interface of a new implant and the enamel powder is suitable for the upper part of the new implant. In maxillary sinus, enamel powder which may maintain volume over a long time is suitable for the upper part of maxillary sinus which is an empty space, and the dentin powder is suitable for the interface of implants contacted with alveolar.

The osteoinduction of dentine powder and the osteoconduction of enamel powder will be described in detail below.

Dentine powder includes about 10 % of hydroxyl apatite (HA) which is an inorganic substance, about 35 % of collagen fiber, and about 5 % of protein (BMP) which are organic substances.

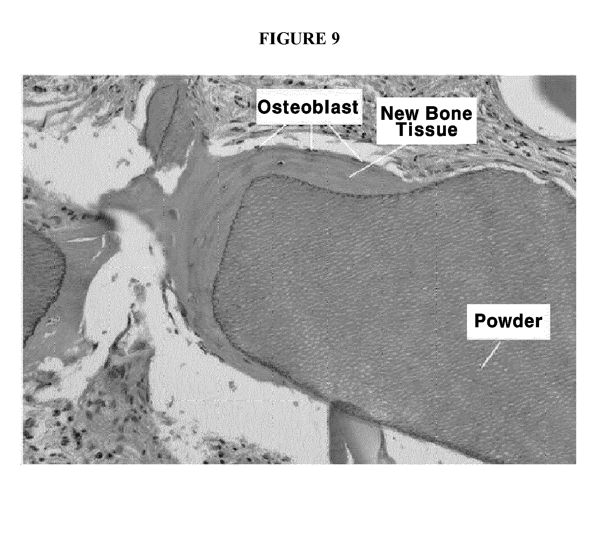

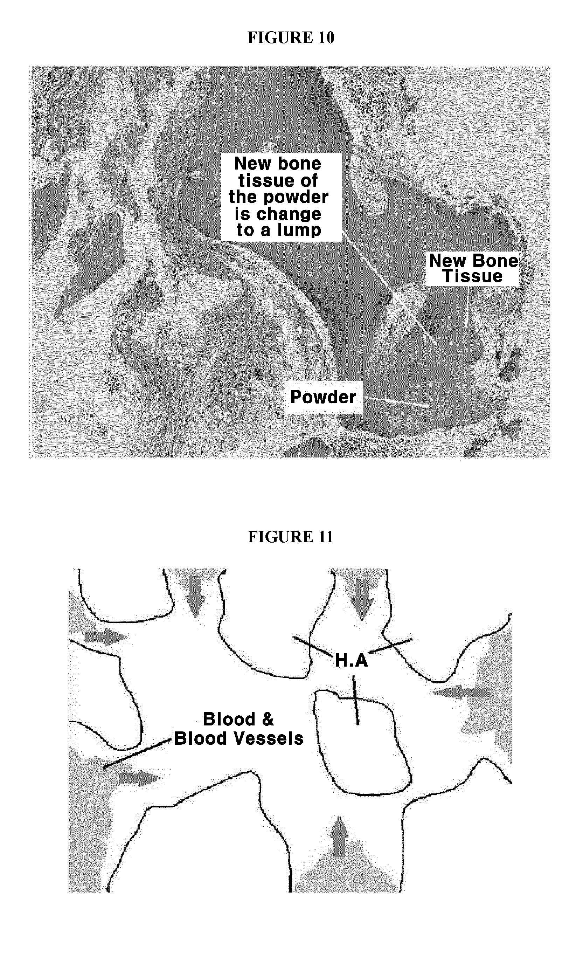

In the course of osteoinduction of dentin powder, firstly, blood vessels and blood are introduced into the dentine powder used as the bone graft material, as shown in FIG. 8. The blood vessel cells are transformed into osteoblast cells by the collagen and the protein of the bone graft material to generate new bone tissue which is adsorbed on the lump of collagen immediately. FIG. 9 shows generation of osteoblast cells and new bone tissue by dentin powder. Then, the new bone tissue and the dentin powder are transformed into a lump of bone through bone remodeling as shown on Fig. 10.

As described above, dentin powder accelerates generation of new bone tissues by osteoinduction and the powder which is the graft material may be replaced by bone within a short period.

The enamel powder has osteoconduction including the introduction of blood and blood vessels, replacement by new bone tissue and adhesion, in the said order, and includes about 96 % of hydroxyl apatite which is an inorganic substance and about 1% to about 2 % of collagen which is an organic substance.

As such, when enamel powder is used as a bone graft material, blood and blood vessels are introduced into the enamel powder, as shown in FIG. 11, and the blood vessels and soft tissues introduced are replaced by new bone tissues, and the new bone tissues and inorganic lump (HA) are adhered.

As described above, the enamel powder keeps inorganic substances longer and the new bone remodeling is slower than the dentine powder, it may maintain volume and shape over a longer time than osteoinduction.

The bone graft materials in accordance with the present invention providing above described features may be produced from the tooth extracted from the patient himself/herself, implanted into alveolar safely without immunity rejection response to generate and maintain alveolar.

Furthermore, the powder produced from a tooth extracted from another patient also may be implanted into alveolar safely by eliminating immune rejection substances.

Therefore, the extracted teeth which have been disposed of may be processed into bone graft materials having the same functionalities and effectiveness as those of autogenous bones, improving the safety of alveolar implantation greatly.

## Claims

1. A method for processing a bone graft material using a tooth, the method comprising steps of:
(a) cutting a tooth extracted from a patient at the boundary between the dental crown and the dental root of the tooth;
(b) removing the soft tissue of the dental root cut;
(c) quick-freezing the dental root and pulverizing the same to prepare a powder having an average diameter of 200 µm to 1,500 µm;
(d) degreasing the powder;
(e) deliming the powder;
(f) dehydrating the powder;
(g) degreasing the powder once again; and
(h) sterilizing the degreased and dehydrated powder to prepare a bone graft material,
and further comprising the step of mixing an additive obtained from the dental crown and having higher osteoconduction with the powder before the step (h).

2. The method of claim 1, wherein the deliming step (e) is performed using 0.1 N to 1.0 N hydrochloric acid aqueous solution having a volume of 5 times to 10 times that of the powder for 10 minutes to 1 hour.

3. The method of claim 1, wherein the additive comprises powder produced from the dental crown, whose soft tissue is removed, by dehydrating and degreasing.

4. The method of claim 1, further comprising a step of washing the dental root with distilled water for 30 minutes to 2 hours after the step (b).

5. The method of claim 1, wherein the quick-freezing process of the step (c) is performed to freeze the dental root in liquid nitrogen at a temperature between -160°C to -210°C for 30 minutes to 2 hours.

6. The method of claim 1, further comprising a step of washing the powder obtained in the step (c) with distilled water for 30 minutes to 2 hours to remove contaminants and residual soft tissues.

7. The method of claim 1, wherein the powder of the step (c) is processed by:
a first washing step where the powder is processed with ultrasonic cleaning for 5 minutes to 10 minutes in pure water or sterilized distilled water;
a second washing step where the powder is processed with ultrasonic cleaning for 10 minutes to 30 minutes in hydrogen peroxide solution; and
a third washing step where the powder is processed with ultrasonic cleaning for 5 minutes to 10 minutes in pure water or sterilized distilled water,
wherein the above processes for three cycles are successively repeated,
wherein the concentration of the hydrogen peroxide solution is 5% to 7%,
the volume of the washing fluid of each washing step is between 5 times to 10 times that of the powder, and
the temperature of the washing fluid is between about 60°C to about 80°C.

8. The method of claim 1, wherein the degreasing steps (d) and (g) are performed using chloroform methanol solution where the weight ratio between the chloroform and methanol is 1:0.5 to 1:2, for 3 hours to 12 hours.

9. The method of claim 1, further comprising:
a step of removing floating fat from the powder by centrifugal separation after degreasing of the step (d); and
a step of washing the powder with distilled water for about one to two hours after the centrifugal separation.

10. The method of claim 1, wherein the dehydrating step (f) is performed for 30 minutes to 2 hours using neutral ethyl alcohol.

11. A bone graft material which is processed with a method of any one of claims 1 to 10.

## Patentansprüche

1. Verfahren zum Bearbeiten eines Knochenersatzmaterials unter Verwendung eines Zahns, wobei das Verfahren die Schritte umfasst:
(a) Schneiden eines von einem Patienten extrahierten Zahns an der Grenze zwischen der Zahnkrone und der Zahnwurzel des Zahns;
(b) Entfernen des Weichgewebes von der geschnittenen Zahnwurzel;
(c) Schockgefrieren der Zahnwurzel und Pulverisieren derselben, um ein Pulver mit einem mittleren Durchmesser von 200 µm bis 1500 µm herzustellen;
(d) Entfetten des Pulvers;
(e) Entkalken des Pulvers;
(f) Dehydratisieren des Pulvers;
(g) noch einmal Entfetten des Pulvers; und
(h) Sterilisieren des entfetteten und dehydratisierten Pulvers, um ein Knochenersatzmaterial herzustellen;
und außerdem den Schritt des Vermischens eines Additivs, das aus der Zahnkrone erhalten wird und eine höhere Osteokonduktion aufweist, mit dem Pulver vor dem Schritt (h) umfasst.

2. Verfahren nach Anspruch 1, wobei der Entkalkungsschritt (e) unter Verwendung einer 0,1 N bis 1,0 N wässrigen Chlorwasserstoffsäurelösung mit einem Volumen vom Fünffachen bis Zehnfachen des Volumens des Pulvers während 10 Minuten bis 1 Stunde durchgeführt wird.

3. Verfahren nach Anspruch 1, wobei das Additiv Pulver umfasst, das aus der Zahnkrone, deren Weichgewebe entfernt ist, durch Dehydratisieren und Entfetten hergestellt wird.

4. Verfahren nach Anspruch 1, das außerdem einen Schritt des Waschens der Zahnwurzel mit destilliertem Wasser während 30 Minuten bis 2 Stunden nach dem Schritt (b) umfasst.

5. Verfahren nach Anspruch 1, wobei der Schockgefrierarbeitsgang des Schritts (c) durchgeführt wird, um die Zahnwurzel in flüssigem Stickstoff bei einer Temperatur zwischen -160 °C bis -210 °C während 30 Minuten bis 2 Stunden einzufrieren.

6. Verfahren nach Anspruch 1, das außerdem einen Schritt des Waschens des Pulvers, das in dem Schritt (c) erhalten wird, mit destilliertem Wasser während 30 Minuten bis 2 Stunden umfasst, um Kontaminanten und restliche Weichgewebe zu entfernen.

7. Verfahren nach Anspruch 1, wobei das Pulver des Schritts (c) bearbeitet wird durch:
einen ersten Waschschritt, bei dem das Pulver mit Ultraschallreinigung während 5 Minuten bis 10 Minuten in reinem Wasser oder sterilisiertem destillierten Wasser bearbeitet wird;
einen zweiten Waschschritt, bei dem das Pulver mit Ultraschallreinigung während 10 Minuten bis 30 Minuten in Wasserstoffperoxidlösung bearbeitet wird; und
einen dritten Waschschritt, bei dem das Pulver mit Ultraschallreinigung während 5 Minuten bis 10 Minuten in reinem Wasser oder sterilisiertem destillierten Wasser bearbeitet wird,
wobei die vorstehenden Arbeitsgänge für drei Zyklen nacheinander wiederholt werden,
wobei die Konzentration der Wasserstoffperoxidlösung 5 % bis 7 % beträgt,
das Volumen der Waschflüssigkeit von jedem Waschschritt zwischen dem Fünffachen bis Zehnfachen des Volumens des Pulvers beträgt, und
die Temperatur der Waschflüssigkeit zwischen ungefähr 60 °C bis ungefähr 80 °C beträgt.

8. Verfahren nach Anspruch 1, wobei die Entfettungsschritte (d) und (g) unter Verwendung einer Chloroform-Methanol-Lösung während 3 Stunden bis 12 Stunden durchgeführt werden, wobei das Gewichtsverhältnis zwischen dem Chloroform und Methanol 1:0,5 bis 1:2 beträgt.

9. Verfahren nach Anspruch 1, das außerdem umfasst:
einen Schritt des Entfernens von schwimmendem Fett von dem Pulver durch Zentrifugalabscheidung nach dem Entfetten des Schritts (d); und
einen Schritt des Waschens des Pulvers mit destilliertem Wasser während ungefähr einer bis zwei Stunden nach der Zentrifugalabscheidung.

10. Verfahren nach Anspruch 1, wobei der Dehydratisierungsschritt (f) während 30 Minuten bis 2 Stunden unter Verwendung von neutralem Ethylalkohol durchgeführt wird.

11. Knochenersatzmaterial, welches mit einem Verfahren nach einem der Ansprüche 1 bis 10 bearbeitet wird.

## Revendications

1. Procédé de traitement d'un matériau pour greffe osseuse utilisant une dent, le procédé comprenant les étapes suivantes :
(a) la coupure d'une dent extraite d'un patient à la limite entre la couronne dentaire et la racine dentaire de la dent ;
(b) l'élimination du tissu mou de la coupure de la racine dentaire ;
(c) la congélation rapide de la racine dentaire et sa pulvérisation pour préparer une poudre ayant un diamètre moyen de 200 µm à 1500 µm ;
(d) le dégraissage de la poudre ;
(e) le déchaulage de la poudre ;
(f) la déshydratation de la poudre ;
(g) le dégraissage de la poudre une fois encore ; et
(h) la stérilisation de la poudre dégraissée et déshydratée pour préparer un matériau pour greffe osseuse,
et comprenant en outre l'étape de mélange d'un additif obtenu à partir de la couronne dentaire et présentant une ostéoconduction supérieure avec la poudre avant l'étape (h).

2. Procédé selon la revendication 1, dans lequel l'étape de déchaulage (e) est réalisée en utilisant une solution aqueuse 0,1 N à 1,0 N d'acide chlorhydrique ayant un volume de 5 fois à 10 fois celui de la poudre pendant 10 minutes à 1 heure.

3. Procédé selon la revendication 1, dans lequel l'additif comprend la poudre produite à partir de la couronne dentaire, dont le tissu mou est éliminé, par déshydratation et dégraissage.

4. Procédé selon la revendication 1, comprenant en outre une étape de lavage de la racine dentaire avec de l'eau distillée pendant 30 minutes à 2 heures après l'étape (b).

5. Procédé selon la revendication 1, dans lequel le procédé de congélation rapide de l'étape (c) est réalisé pour congeler la racine dentaire dans de l'azote liquide à une température située entre -160 °C et -210 °C pendant 30 minutes à 2 heures.

6. Procédé selon la revendication 1, comprenant en outre une étape de lavage de la poudre obtenue dans l'étape (c) avec de l'eau distillée pendant 30 minutes à 2 heures pour éliminer les contaminants et les tissus mous résiduels.

7. Procédé selon la revendication 1, dans lequel la poudre de l'étape (c) est traitée par :
une première étape de lavage où la poudre est traitée par nettoyage aux ultrasons pendant 5 minutes à 10 minutes dans de l'eau pure ou de l'eau distillée stérilisée ;
une deuxième étape de lavage où la poudre est traitée par nettoyage aux ultrasons pendant 10 minutes à 30 minutes dans une solution de peroxyde d'hydrogène ; et
une troisième étape de lavage où la poudre est traitée par nettoyage aux ultrasons pendant 5 minutes à 10 minutes dans de l'eau pure ou de l'eau distillée stérilisée,
dans lequel les procédés ci-dessus sont répétés successivement pendant trois cycles,
dans lequel la concentration de la solution de peroxyde d'hydrogène est de 5 % à 7 %,
le volume du liquide de lavage de chaque étape de lavage se situe entre 5 fois et 10 fois celui de la poudre, et
la température du liquide de lavage se situe entre environ 60 °C et environ 80 °C.

8. Procédé selon la revendication 1, dans lequel les étapes de dégraissage (d) et (g) sont réalisées en utilisant une solution de chloroforme/méthanol où le rapport pondéral entre le chloroforme et le méthanol est de 1/0,5 à 1/2, pendant 3 heures à 12 heures.

9. Procédé selon la revendication 1, comprenant en outre :
une étape d'élimination de la graisse flottante à partir de la poudre par séparation centrifuge après le dégraissage de l'étape (d) ; et
une étape de lavage de la poudre avec de l'eau distillée pendant environ une à deux heures après la séparation centrifuge.

10. Procédé selon la revendication 1, dans lequel l'étape de déshydratation (f) est réalisée pendant 30 minutes à 2 heures en utilisant de l'alcool éthylique neutre.

11. Matériau pour greffe osseuse qui est traité par un procédé selon l'une quelconque des revendications 1 à 10.
